(19) Europäisches Patentamt
European Patent Office
Office européen des brevets

(11) EP 4 589 297 A1

(12) **EUROPEAN PATENT APPLICATION**
published in accordance with Art. 153(4) EPC

(43) Date of publication:
**23.07.2025 Bulletin 2025/30**

(21) Application number: **23879882.1**

(22) Date of filing: **19.10.2023**

(51) International Patent Classification (IPC):
***G01N 33/53*** (2006.01) ***G01N 33/543*** (2006.01)

(52) Cooperative Patent Classification (CPC):
**G01N 33/53; G01N 33/543**

(86) International application number:
**PCT/JP2023/037939**

(87) International publication number:
**WO 2024/085239 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2022 JP 2022169526**

(71) Applicants:
- **Unicharm Corporation**
  **Shikokuchuo-shi, Ehime 799-0111 (JP)**
- **Cranebio Co., Ltd.**
  **Tokyo, 101-0021 (JP)**

(72) Inventors:
- **SUZUKI, Yuya**
  **Kanonji-shi, Kagawa 769-1602 (JP)**
- **NOMOTO, Takashi**
  **Kanonji-shi, Kagawa 769-1602 (JP)**
- **HASHINO, Akira**
  **Kanonji-shi, Kagawa 769-1602 (JP)**
- **SAITO, Keita**
  **Tokyo 101-0021 (JP)**
- **SUZUKI, Takeru**
  **Tokyo 101-0021 (JP)**
- **TAKESHITA, Moto**
  **Tokyo 101-0021 (JP)**
- **ABE, Misato**
  **Tokyo 101-0021 (JP)**
- **SUGAWARA, Iku**
  **Tokyo 101-0021 (JP)**
- **HAYASHI, Risako**
  **Kyoto-shi, Kyoto 604-8845 (JP)**
- **CHIBA, Yoko**
  **Kyoto-shi, Kyoto 604-8845 (JP)**

(74) Representative: **Dolleymores**
**9 Rickmansworth Road**
**Watford, Hertfordshire WD18 0JU (GB)**

(54) **TEST STRIP FOR VAGINAL DISCHARGE**

(57) Provided is a test strip which can be used as a new tool for ovulation date measurement, wherein the test strip is for detecting luteinizing hormone in vaginal discharge using immunochromatography, and wherein 0.05 mIU/mL to 2.5 mIU/mL of luteinizing hormone is detected as positive.

Fig. 4

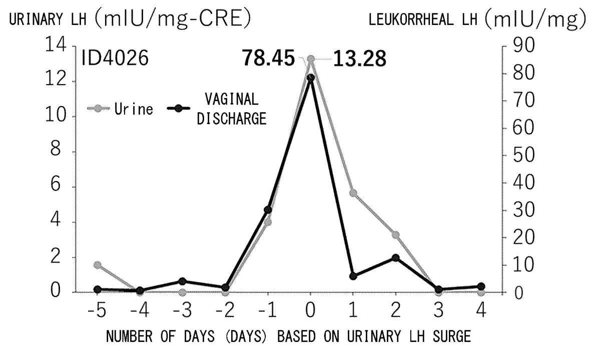


LEUKORRHEAL LH AND URINARY LH SURGED ON THE SAME DAY

EP 4 589 297 A1

## Description

FIELD

The present invention relates to a vaginal discharge test strip.

BACKGROUND

Identifying the day of ovulation in advance is important for women hoping to become pregnant. Conventionally, methods for testing luteinizing hormone (LH) in urine and kits therefor have been widely used as a method for predicting the day of ovulation.

Lateral flow assays for detecting the presence of an analyte in a liquid sample are also widely known (Patent Literature 1).

[CITATION LIST]

[PATENT LITERATURE]

[PTL 1] Japanese Unexamined PCT Publication (Kohyo) No. 2007-526443

SUMMARY

[TECHNICAL PROBLEM]

An object of the present invention is to provide a test strip which can be used as a new tool for predicting the day of ovulation.

[SOLUTION TO PROBLEM]

The object of the present invention can be achieved by the present invention, including the following aspects.

<Aspect 1>

A test strip for detecting luteinizing hormone in vaginal discharge using immunochromatography, wherein 0.05 mIU/mL to 2.5 mIU/mL of luteinizing hormone is detected as positive.

<Aspect 2>

The test strip according to Aspect 1, wherein the test strip has a width of 5 mm or less.

<Aspect 3>

The test strip according to Aspect 1, wherein the test strip has a width of 3 mm or less.

<Aspect 4>

The test strip according to any one of Aspects 1 to 3, wherein the test strip comprises:

a first member which contacts vaginal discharge,
a second member which contains a labeled antibody that recognizes luteinizing hormone in vaginal discharge, and
a third member which has a display part which contains a capture antibody,
the capture antibody is capable of capturing a complex formed by binding of the labeled antibody and luteinizing hormone, and
the test strip is configured such that the luteinizing hormone in vaginal discharge moves from the first member, through the second member, to the display part of the third member, and that
the display part of the third member changes color when the capture antibody captures the complex.

<Aspect 5>

The test strip according to Aspect 4, wherein the first member, the second member, and the third member are stacked in this order at least partially overlapping each other, and
a sum of thicknesses of the first member, the second member, and the third member in the stacking direction is 1.5 mm or less.

<Aspect 6>

The test strip according to Aspect 5, for use in direct contact with the vagina.

<Aspect 7>

The test strip according to any one of Aspects 4 to 6, which is configured such that a top side and a back side of the test strip can be visually distinguished.

<Aspect 8>

The test strip according to any one of Aspects 4 to 7, wherein the first member is composed of fibers having an average fiber diameter of 0.5 to 5 μm,

the second member is composed of fibers having an average fiber diameter of 10 to 40 μm, and
an average fiber diameter of the fibers of the first member is less than an average fiber diameter of the fibers of the second member.

<Aspect 9>

The test strip according to any one of Aspects 4 to 8, wherein an average fiber diameter R1 of the fibers constituting the first member and an average fiber diameter R2 of the fibers constituting the second member satisfy the relationship $5 \leq (R2/R1) \leq 20$.

<Aspect 10>

The test strip according to any one of Aspects 4 to 9, which satisfies at least one of the following:

(a) the second member has a basis weight of 100 gsm or more,
(b) a sum of the thicknesses of the first member, the second member, and the third member is 1.5 mm or less, and the thickness of the second member accounts for 30% or more of this sum of the thicknesses, and
(b) the second member has a density of 0.05 to 0.80 g/$m^3$.

<Aspect 11>

The test strip according to any one of Aspects 4 to 10, wherein the labeled antibody comprises a colorant having a particle diameter of 10 nm to 500 nm.

<Aspect 12>

The test strip according to any one of Aspects 4 to 10, wherein the labeled antibody comprises a colorant having a particle diameter of 250 nm to 500 nm.

<Aspect 13>

The test strip according to any one of Aspects 4 to 12, wherein a surfactant is applied to the first member, which is a sample pad.

<Aspect 14>

The test strip according to any one of Aspects 4 to 13, wherein the first member, which is a sample pad, contains a boron compound and a lectin.

[ADVANTAGEOUS EFFECTS OF THE INVENTION]

According to the present invention, a test strip which can be used as a new tool for predicting the day of ovulation can be provided.

BRIEF DESCRIPTION OF DRAWINGS

FIG. 1 is a photograph showing the results of a sensitivity test of test strips according to the present invention.
FIG. 2 is a photograph showing the results of a color intensity test of test strips according to the present invention.
FIG. 3-1 is a schematic view showing a specific configuration of a test strip according to the present invention.
FIG. 3-2 is a conceptual diagram showing a specific configuration of a test strip according to the present invention.
FIG. 4 is a graph plotting urinary LH concentration and leukorrheal LH concentration relative to the number of days based on the urinary LH surge.
FIG. 5 is a graph plotting urinary LH concentration and leukorrheal LH concentration relative to the number of days based on the urinary LH surge.
FIG. 6 is a graph showing LH concentration around the day of leukorrheal LH surge.
FIG. 7 is a graph showing the results of ROC analysis.

Since the present test strip can reliably detect LH in vaginal discharge, it can be used as a new tool for predicting the day of ovulation. Specifically, for example, it can be used as a biomarker for detecting LH surge using vaginal discharge as a specimen.

DESCRIPTION OF EMBODIMENTS

The details of the present invention will be described below.

<Aspect 1>

A test strip for detecting luteinizing hormone in vaginal discharge using immunochromatography, wherein 0.05 mIU/mL to 2.5 mIU/mL of luteinizing hormone is detected as positive.

As described above, in conventional methods for predicting the day of ovulation, urine is generally used as the detection target. Conversely, the present inventors have focused on vaginal discharge, which is a substance discharged from the vagina, to develop a new tool for predicting the day of ovulation.

There has been little knowledge regarding luteinizing hormone in vaginal discharge to date. Furthermore, vaginal discharge has a relatively high viscosity and low fluidity. Further, the sample volume of vaginal discharge is small compared to urine, etc. Thus, it has been difficult in some cases to detect LH in vaginal discharge by conventional methods, and in particular, in detection using immunochromatography.

In light of this background, the present inventors have discovered that according to the present test strip for detecting 0.05 mIU/mL to 2.5 mIU/mL of luteinizing hormone as positive, luteinizing hormone in vaginal discharge can be reliably detected, and the day of ovulation can be reliability predicted. Thus, according to the test strip according to Aspect 1, a test strip which can be used as a new tool for predicting the day of ovulation can be provided.

The test strip preferably detects 0.05 mIU/mL to 2.0 mIU/mL of luteinizing hormone as positive.

As will be easily understood by a person skilled in the art, "detects 0.05 mIU/mL to 2.5 mIU/mL of luteinizing hormone (LH) as positive" means that a positive result is indicated when the LH concentration is within this concentration range, but does not mean that a positive result is indicated "only" when the LH concentration is within the concentration range. Specifically, for example, a test strip which indicates a positive result for LH in the concentration range of 0.05 mIU/mL to 10.0 mIU/mL indicates a positive result for LH in the concentration range of 0.05 mIU/mL to 2.5 mIU/mL, and is thus included in the scope of the present invention.

An important point of the present invention is that, unlike conventional urinary LH detectors, the test strip is configured to detect LH within a relatively low concentration range of 0.05 mIU/mL to 2.5 mIU/mL as positive.

From the viewpoint of improving the reliability of detection, it is preferable that the test strip not detect as positive at concentrations in the range of less than 0.001 mIU/mL, and particularly preferably not detect as positive at concentrations in the range of less than 0.005 mIU/mL, less than 0.01 mIU/mL, less than 0.02 mIU/mL, less than 0.03 mIU/mL, less than 0.04 mIU/mL, or less than 0.05 mIU/mL.

The test strip according to Aspect 1 described above can detect as positive, for example, by color change that occurs when the luteinizing hormone is within the above concentration range.

Furthermore, in the test strip according to Aspect 1 described above, for example, when the luteinizing hormone is within the above-mentioned concentration range (for example, 0.05 mIU/mL to 2.5 mIU/mL), color change can occur at a level lower than the maximum level. The level of color change can be visually observed, for example.

A test strip capable of detecting LH within the above concentration range as positive can be produced by, for example, adjusting the sensitivity (for example, colorant) of a substance (for example, antibody) which reacts with luteinizing hormone, and in particular, by adjusting the size of a colorant adhering to the antibody which recognizes luteinizing hormone.

The test strip detects:

more preferably, 0.05 mIU/mL to 1.0 mIU/mL.
further preferably, 0.05 mIU/mL to 0.5 mIU/mL, and
even further preferably, 0.05 mIU/mL and 0.2 mIU/mL of luteinizing hormone as positive.

The detection concentration of the test strip can be measured using a casein blocking buffer solution of LH as a standard solution.

The casein blocking buffer solution of LH can be prepared, for example, in the following manner:

(a) A blocking buffer is produced by dissolving a casein blocking agent (1.0% casein, 100 mM borate, pH 8.5) in purified water.
(b) A standard LH solution of known concentration is diluted with the blocking buffer to produce an LH standard solution.

The casein blocking agent can be prepared, for example, as follows:
(1.0% Casein, 100 mM Borate, pH 8.5)

(a) Add 800 g of distilled water to a 1000 ml beaker.
(b) Add 2.0 ml of 20% NaOH.
(c) Stir for 10 minutes.
(d) Slowly add 10.0 g of casein powder.
(e) Stir for 3 hours.
(f) Add 6.18 g (= 100 mM) of boric acid.
(g) Stir for 10 minutes.
(h) Add 2.5 ml of 20% NaOH.
(i) Dilute to 1000 ml with distilled water.
(j) Stir for 10 minutes.
(k) Measure the pH.
(l) Filter using a filter unit (pore size 0.45 $\mu$m).
(m) Heat-treat at 30°C for 24 hours.
(n) Dispense into 15 ml or 50 ml containers for storage.
(o) Store at -80°C.

Conventional urinary LH testing methods are intended to detect a much higher concentration range as positive than the concentration range of 0.05 mIU/mL to 2.5 mIU/mL according to the present invention. Though it is possible to sample and detect LH in blood, this method is not convenient since drawing blood is painful and causes bodily injury at the time of sampling.

For example, according to the prior art (Bioeng. Transl. Med., 2017, 2(3), pp. 238-246), the natural LH surge concentration in urine is 20 to 100 mIU/ml.

Conventional luteinizing hormone kits using urine specimen have a urinary luteinizing hormone concentration of 20 mIU/mL to 100 mIU/mL as a detection sensitivity range (Notification No. 0222-1, February 22, 2016, issued by the Director of Medical Device Evaluation Division, Pharmaceutical Safety and Environmental Health Bureau, Ministry of Health, Labor and Welfare).

The following literature, which concerns urinary and blood hormones, relates to a study of reproductive hormone levels in urine during the menstrual cycle, and describes that the median total LH value was 38.3 (mIU/ml) on the day before ovulation (Table 2):
"Monitoring the Menstrual Cycle: Comparison of Urinary and Serum Reproductive Hormones Referenced True Ovulation", The European Journal of Contraception and Reproductive Health Care, 2015; Early Online: 1-13.

The following literature is a document on urinary and blood hormones, and describes the LH reference range during the ovulation phase of a woman as 8 to 100 (mIU/mL):
"Kansai Medical University - List of Endocrine Reference Values"
The following literature lists a baseline LH value of 6.69 (±5.22) (mIU/mg Cr) and a peak LH value of 41.2 (±20.0) (mIU/mg Cr):
"Characteristics of Urinary Luteinizing Hormone Surge in Young Ovulatory Women" Fertil. Steril. 2007 Sep; 88(3); 684-90

<Aspect 2>

In an aspect (Aspect 2) of the test strip according to the present disclosure, the test strip has a width of 5 mm or less.
The width of the test strip may be 1 mm to 10 mm, but is preferably 5 mm or less. The upper limit of the width of the test strip may further be 4 mm or less. The width of the test strip is particularly preferably 1 mm or more and less than 5 mm, 1.5 mm to 4.5 mm, or further preferably 2 mm to 4 mm. When the width of the test strip is within these ranges, particularly suitable visibility of the color reaction, etc., may be obtained.

<Aspect 3>

In an aspect of the test strip according to the present disclosure, the test strip has a width of 3 mm or less.
From the viewpoints of visibility for the user and ease of processing, the width of the test strip is preferably 2 mm or more, and particularly preferably 2 to 3 mm.
In the test strip according to Aspects 2 and 3, since the width of the test strip is reduced to 5 mm or less, or further, 3 mm or less, the amount of liquid that needs to be retained for detection is relatively reduced. Thus, in the test strip according to Aspect 2 or 3, even when the detection target is a vaginal discharge which is small in volume, has a high viscosity, and has a small amount of LH, LH can suitably be detected.

<Aspect 4>

In an aspect of the test strip according to the present disclosure, the test strip comprises:

a first member which contacts vaginal discharge,
a second member which contains a labeled antibody that recognizes luteinizing hormone in vaginal discharge, and
a third member which has a display part which contains a capture antibody,
the capture antibody is capable of capturing a complex formed by binding of the labeled antibody and luteinizing hormone, and
the test strip is configured such that the luteinizing hormone in vaginal discharge moves from the first member, through the second member, to the display part of the third member, and that
the display part of the third member changes color when the capture antibody captures the complex.

According to the test strip described in Aspect 4, luteinizing hormone can be detected in a convenient manner and quickly via a color reaction.
The measurement principle of the test strip will be described using a specific example. In an exemplary aspect, for example, the labeled antibody is a colorant-labeled mouse monoclonal antibody against human luteinizing hormone, and the capture antibody is a mouse monoclonal antibody against human luteinizing hormone. If human luteinizing hormone (hLH) is present in a specimen which has contacted the first member, the labeled antibody reacts with the hLH in the second member to form a complex based on the antigen-antibody reaction. This complex moves to the display part of the third member and is captured by the capture antibody immobilized and bound there, and as a result, the display part changes color based on the colorant (pigment).
A second display part (control part) can be provided downstream of the display part in the direction of antigen movement. A second capture antibody (for example, rabbit anti-mouse immunoglobulin polyclonal antibody) different from the capture antibody described above is immobilized and bound to this control part. This second capture antibody can recognize the labeled antibody. Labeled antibodies which do not form a complex with the antigen pass through the display part without being captured by the display part, and are captured by the control part, resulting in the control part changing color based on a colorant.
In this aspect, in the case of a positive result, the display part and the control part change color, and in the case of a negative result, only the control part changes color.
The configuration of the test strip will be described in detail later.

<Aspect 5>

In an aspect of the test strip according to the present disclosure, the first member, the second member, and the third member are stacked in this order at least partially overlapping each other, and
a sum of thicknesses of the first member, the second member, and the third member in the stacking direction is 1.5 mm or less.

As described above, vaginal discharge has a relatively high viscosity and low fluidity. Further, vaginal discharge is present in smaller amounts compared to urine. Thus, it has sometimes been difficult to detect LH in vaginal discharge by conventional methods, in particular, in detection using immunochromatography.

The present inventors have discovered that the amount of LH in vaginal discharge is smaller than that in urine. In conventional test strips, it has been difficult to detect LH in vaginal discharge.

In the test strip according to Aspect 5, since the thickness of the members constituting the test strip is reduced, the amount of liquid which needs to be retained for detection is relatively reduced. Thus, in the test strip according to Aspect 5, even when the detection target is vaginal discharge, which is small in volume, has high viscosity, and contains a small amount of LH, LH can suitably be detected.

The thickness of each member (membrane thickness) is preferably in the range of 90 to 230 μm, and more preferably 100 to 220 μm. In particular, the thickness of each member (membrane thickness) is preferably in the range of 160 to 230 μm, and more preferably 180 to 220 μm.

<Aspect 6>

An aspect of the test strip according to the present disclosure provides the test strip according to Aspect 5, for use in direct contact with the vagina.

When the test strip is used in direct contact with the vagina, the user can obtain the convenience of confirming the detection results by themselves. However, since the area is difficult to visually confirm, there is a risk of damage to the mucous membrane when using it by feel.

Conversely, the invention of Aspect 5 enables the direct sampling of LH from excreted vaginal discharge from the vagina while ensuring accuracy, and has a relatively thin structure, reducing the risk of damage to the mucous membrane.

<Aspect 7>

In an aspect (Aspect 7) of the test strip according to the present disclosure, the test strip is configured such that a top side and a back side of the test strip can be visually distinguished. According to this, since the user of the test strip can determine which side of the strip the vaginal discharge should be applied to, misuse and incorrect detection can be prevented.

<Aspect 8>

In an aspect (Aspect 8) of the test strip according to the present disclosure,

the first member is composed of fibers having an average fiber diameter of 0.5 to 5 μm (or a fineness of 0.01 to 0.1 dtex),
the second member is composed of fibers having an average fiber diameter of 10 to 40 μm (or a fineness of 2 to 6 dtex), and
an average fiber diameter of the fibers of the first member is less than an average fiber diameter of the fibers of the second member.

As described above, vaginal discharge has a relatively high viscosity and low fluidity. Thus, it has sometimes been difficult to detect LH in vaginal discharge, in particular, in detection using immunochromatography. Further, the present inventors discovered that the amount of LH in vaginal discharge is smaller than that in urine.

In the test strip according to Aspect 8, since the average fiber diameters of the members constituting the test strip are optimized, even vaginal discharge, which is present in a small amount and is highly viscous, is allowed to efficiently move to the detection area (display part), whereby detection can suitably be carried out.

The first member is preferably composed of fibers having an average fiber diameter of 1 to 4 μm, and more preferably an average fiber diameter of 1.5 to 3 μm.

The first member is preferably composed of fibers having a fineness of 0.02 to 0.08 dtex, and more preferably 0.03 to 0.06 dtex.

The second member is preferably composed of fibers having an average fiber diameter of 15 to 30 μm, and more

preferably an average fiber diameter of 18 to 25 $\mu$m.
The second member is preferably composed of fibers having a fineness of 2.5 to 5 dtex, and more preferably a fineness of 3 to 5 dtex.
The average fiber diameter of the fibers of the first member is preferably smaller than the average fiber diameter of the fibers of the second member, which can further improve the absorption by the first member, in particular, of relatively viscous vaginal discharge.
The average fiber diameters of the fibers constituting the first member and the second member can be measured by a scanning electron microscope, and the average fiber diameter can be the average value of the fiber diameters measured for N=30 or more fibers. Note that when measuring in a cross section, care should be taken because the shapes of the fibers may have changed when they were cut. Further, when measuring in a planar direction, since there may be cases in which the fibers have been fused, measurement should be performed avoiding portions where the shape has changed, such as fused parts.

<Aspect 9>

In an aspect of the test strip according to the present disclosure, an average fiber diameter R1 of the fibers constituting the first member and an average fiber diameter R2 of the fibers constituting the second member satisfy the relationship $5 \leq (R2/R1) \leq 20$.
According to this aspect, the relationship between the average fiber diameters of the first and second members constituting the test strip is optimized, and as a result, even vaginal discharge which is present in a small amount and is highly viscous is allowed to efficiently move to the detection area (display part), whereby detection can suitably be carried out. Specifically, the first member has improved absorbency, particularly for highly viscous vaginal discharge. Further, the effect of improved supporting rate of the labeled antibody in the second member is also obtained.
In a more preferable aspect, the following are satisfied:

$$6 \leq (R2/R1) \leq 18$$

$$7 \leq (R2/R1) \leq 16$$

$$8 \leq (R2/R1) \leq 14$$

$$9 \leq (R2/R1) \leq 12$$

<Aspect 9-2>

In an aspect of the test strip according to the present disclosure, a fineness D1 of the fibers constituting the first member and a fineness D2 of the fibers constituting the second member satisfy the relationship $50 \leq (D2/D1) \leq 200$.
According to this aspect, since the relationship between the finenesses of the first and second members constituting the test strip is optimized, even vaginal discharge which is present in a small amount and is highly viscous is allowed to efficiently move to the detection area (display part), whereby detection can suitably be carried out.
In a more preferable aspect, the following are satisfied:

$$60 \leq (D2/D1) \leq 190$$

$$70 \leq (D2/D1) \leq 180$$

$$80 \leq (D2/D1) \leq 170$$

$$90 \leq (D2/D1) \leq 160$$

<Aspect 10>

The present disclosure includes the test trip according to any of Aspects 3 to 9, which satisfies at least one of the following:

(a) the second member has a basis weight of 100 gsm or more,
(b) a sum of the thicknesses of the first member, the second member, and the third member is 1.5 mm or less, and the thickness of the second member accounts for 30% or more of this sum of the thicknesses, and
(b) the second member has a density of 0.05 to 0.80 g/m$^3$.

According to the invention described in Aspect 10, the basis weight, thickness, and/or density of the second member (conjugate pad) is optimized, thereby improving the diffusibility of the labeled antibody. Thus, the detection can suitably be performed even from vaginal discharge with a relatively high viscosity.

<Aspect 11>

In an aspect (Aspect 11) of the test strip according to the present disclosure, the labeled antibody comprises a colorant having a particle diameter of 10 nm to 500 nm.
When the colorant of the labeled antibody which binds to LH has a particle diameter of 10 nm to 500 nm, sufficient detectability can be secured.
Examples of such colorants include colloidal gold and cellulose nanoparticles. Colloidal gold generally has a particle diameter of approximately 40 to 100 nm.

<Aspect 12>

In an aspect (Aspect 12) of the test strip according to the present disclosure, the labeled antibody comprises a colorant having a particle diameter of 250 nm to 500 nm (preferably 280 to 450 nm, and more preferably 300 nm to 400 nm). Examples of such colorants include cellulose nanoparticles.
As described above, the present inventors have discovered that the amount of LH in vaginal discharge is smaller than that in urine. In this case, it is not easy to detect LH in vaginal discharge using conventional test strips (in particular, those targeting LH in urine).
In the test strip according to Aspect 12, the labeled antibody which binds to LH contains a colorant having a relatively large particle diameter, improving the visibility for detection. Thus, according to the test strip according to Aspect 12, even when the detection target is vaginal discharge which contains a small amount of LH, the LH can suitably be detected.

<Aspect 13>

In an aspect (Aspect 13) of the test strip according to the present disclosure, a surfactant is applied to the first member, which is a sample pad.
When a surfactant is applied to the first member, which is a sample pad, the effect of providing optimal affinity between the target substance (vaginal discharge) and the sample pad can be obtained.

<Aspect 14>

In an aspect (Aspect 14) of the test strip according to the present disclosure, the first member, which is a sample pad, contains a boron compound and a lectin.
According to Aspect 14, inhibition of the antigen-antibody reaction of mucin can be suppressed.

<Specific Examples of Test Strip>

The test strip of the present invention will be specifically described with reference to the drawings. Note that the drawings are not necessarily to scale and are not intended to limit the present invention.
The test strip (test member) 60 of FIG. 3-1 has a display part (62A, 62B) which can change color based on excretions (vaginal discharge). The test strip 60 has a contact part (first member, for example, a sample pad) 64 which contacts the vaginal discharge. The test strip 60 may have a moving part through which components contained in the vaginal discharge (hereinafter, vaginal discharge components) move. In the moving part, the vaginal discharge components may move by capillary action. The moving part may have a display moving part 66A through which the vaginal discharge components move from the contact part 64 to the display part (62A, 62B), and a terminal moving part 66B through which the vaginal discharge components move in a direction away from the display part (62A, 62B). The terminal moving part is a part through which the vaginal discharge components that have passed through the display part (62A, 62B) move.

The contact part (first member) 64 with which the vaginal discharge comes into contact may be composed of, for example, a pad (which may be referred to as a sample pad or specimen pad). The vaginal discharge components in the vaginal discharge which have come into contact with the contact part 64 move, for example, through the pad and migrate to a second member (for example, a conjugate pad). The conjugate pad contains a labeled antibody which recognizes luteinizing hormone in vaginal discharge. Though the conjugate pad (second member) is not illustrated in FIG. 3-1, it may be arranged so as to overlap the sample pad (first member) in the thickness direction. The vaginal discharge components, the complex formed by the antigen-antibody reaction with the luteinizing hormone contained in the vaginal discharge components, and the labeled antibody may move through the moving part 66A by capillary action. The part where these substances move may be composed of, for example, a membrane. The display part has an area 62A containing a capture antibody for capturing (binding) the formed complex. This area changes color when the complex is captured by the capture antibody. This area 62A of the display part may be referred to as a test line.

The display part may have an area 62B containing a capture antibody in addition to the area 62A containing the capture antibody for capturing the complex. The capture antibody of the area 62B may capture a complex other than the complex captured in the area 62A, or may capture the labeled antibody. The area 62B changes color when a complex or the labeled antibody is captured. The area 62B for capturing the labeled antibody may be referred to as a control line. The vaginal discharge components which have passed through the display part 62 (i.e., the components which have not been captured by the capture antibody) move through the terminal moving part 66B. The terminal moving part 66B may be composed of a membrane. The terminal moving part 66B may also have an adsorption pad for absorbing the vaginal discharge components which have passed through the display part 62. The vaginal discharge components which have passed through the display part 62 may be absorbed by the absorbent core instead of the adsorption pad. When immunochromatography is used, the display part 62 and the contact part 64 are arranged at different positions. Thus, in this case, the display part 62 and the contact part 64 are different.

The test strip (test member) 60 needs only to comprise members which are capable of retaining or containing substances such as labeled antibodies and captured antibodies, and may be composed of any of the materials such as paper, nonwoven fabric, or woven fabric.

A conceptual diagram of the test strip is shown in the attached FIG. 3-2. Regarding the other structures of the test strip, reference can be made to descriptions of, for example, WO 2021/132724A1.

<Method for Detecting Luteinizing Hormone from Vaginal Discharge>

The present disclosure includes a method for detecting luteinizing hormone from vaginal discharge using a test strip using immunochromatography, the method comprising the step of:

allowing vaginal discharge to adhere to the test strip, wherein
in the test strip, 0.05 mIU/mL to 2.5 mIU/mL of luteinizing hormone is detected as positive.

Regarding details of each constituent element in this method, such as the test strip, reference can be made to the above descriptions of the test strip.

The method of allowing vaginal discharge to adhere to the test strip is not particularly limited, and reference can be made to the above descriptions regarding the test strip. For example, in the test strip of Aspect 4 described above, vaginal discharge can adhere to the test strip by allowing the vaginal discharge to directly adhere to the first member (in particular, the sample pad). Preferably, the test strip is allowed to be in direct contact with the vagina (vulva).

In an embodiment of the method described above, 0.05 mIU/mL to 1.0 mIU/mL, or 0.05 mIU/mL to 0.5 mIU/mL, or 0.05 mIU/mL to 0.2 mIU/mL of luteinizing hormone is detected as positive.

EXAMPLES

The present invention will be described in detail below with reference to the Examples, but the present invention is not limited to these Examples.

<Experimental Example>

<<Potential of Leukorrhea Luteinizing Hormone (LH) as Biomarker for Predicting Day of Ovulation>>

The use of leukorrhea luteinizing hormone (LH) as a biomarker for predicting the day of ovulation was investigated.

It is important for women who wish to become pregnant (women attempting to conceive) to identify the day of ovulation in advance. From the viewpoint of convenience, the most widely used test kit at the moment is a urinary luteinizing hormone (LH) test kit. Measurement of urinary LH is relatively convenient, as it can be completed in about 15 minutes after applying urine to the test kit. However, daily measurement is a heavy burden on the user, and the utilization rate of urinary LH test kits among women attempting to conceive is not high. Thus, in order to establish a new method of predicting the day of ovulation, we focused on leukorrhea as a measurement specimen. Not only can leukorrhea be sampled non-invasively, but it requires no conscious effort for sampling, making it a specimen that can achieve a "testing" with minimal user burden "where the measurement is completed before the user even realizes it". However, there are few research examples of leukorrhea as a measurement specimen, and the measurement of ovulation prediction biomarkers such as LH therein has not been reported. Thus, we detected leukorrheal LH and examined its potential as a biomarker.

The test methods and results as well as discussion and conclusion are shown below.

<Methods>

•Subject Recruitment

Subjects were women aged 20 years or older and under 40 years old who had no menstrual-related illnesses, and whether they had never been pregnant or had given birth was not an issue.

•Collection of Specimens from Subjects

Subjects were provided with urine specimen sampling kits and unscented panty liners, and urine and the panty liners from the 10th to 19th postmenstrual days were collected. To prevent evaporation of liquid from the panty liners, the panty liners were immediately packed in non-permeable films after removal and stored at -18°C or lower, and then transported at -15°C or lower and collected.

•Extraction of Leukorrhea Component from Panty Liners

The center portion of each of the collected panty liners was cut into a sizer of 2 $cm^2$, which was then immersed in 500 μl of PBS and centrifuged at 3600 rpm to collect the extract, which was used as a measurement specimen.

•Specimen Measurement

LH in urine and in leukorrhea was measured by Enzyme-Linked Immunosorbent Assay (ELISA). In order to exclude external factors influencing the concentration in the specimens, urine was measured based on urinary creatinine (ELISA) and leukorrhea was measured based on total protein amount (BCA assay), and the values were corrected.

•Definition of Leukorrhea Surge

The leukorrheal LH surge was defined as follows:

A concentration increase of at least 2-fold as compared to the previous day, the leukorrheal LH/total protein exceeding 0.05 mIU/mg, wherein
If multiple peaks are present, the earliest peak is used in place of the top peak, assuming an actual test for leukorrheal LH.

•Exclusion Criteria

Specimens which met any of the following conditions were excluded from measurement specimens:

Specimen in which no urinary LH surge appeared during the collection period;
Specimen in which the concentration increased during the collection period, possibly due to urinary LH surge, but the concentration did not decrease during the period; and
Specimen from which leukorrhea could not be collected and the day on which the leukorrheal LH surge started was unknown.

•Count of Specimen Used in Analysis

16 individuals
29 specimens

•Results

(1) FIGS. 4 and 5 are graphs plotting urinary LH concentrations and leukorrheal LH concentrations relative to the number of days based on the urinary LH surge. It is known that the urinary LH surge appears several hours later than the blood LH surge. The leukorrheal LH does not have a fixed relationship with the urinary LH surge. In addition to a pattern in which the leukorrheal LH surge and urinary LH surge were observed on the same day, there was also a pattern in which the leukorrheal LH surge was observed with a time shift before or after the urinary LH surge.
(2) Table 1 shows the day of ovulation anticipated from the urinary LH surge and the ratio of the numbers of specimens in which a leukorrheal LH surge was observed. For the fertile window, the criteria established by Dunson et al. (*2) was used. When the day of ovulation was determined based on the urinary LH surge, the ratio of the number of specimens in which a leukorrheal LH surge was observed appearing in the fertile window totaled 86%.

(*2) D. B. Dunson et al. Day-Specific Probabilities of Clinical Pregnancy Based on Two Studies with Perfect Measurements of Ovulation, Human Reproduction, 1999;14(7): pp. 1835-1839

[Table 1]

| Table 1: Number of days from day of ovulation predicted from urinary LH surge and Ratio of number of specimens in which leukorrheal LH surge was observed | | |
|---|---|---|
| Number of days (Days) from day of ovulation predicted from urinary LH surge | Number of specimens in which leukorrheal LH surge was observed | Ratio |
| -4 | 4 | 14% |
| -3 | 1 | 3% |
| -2 | 8 | 28% |
| -1 | 8 | 28% |
| 0 (day of ovulation) | 4 | 14% |
| Other than above | 4 | 14% |
| Total | 29 | 100% |
| Total 86% | | |

As can be seen from FIGS. 4 to 7, the LH surge in vaginal discharge appeared in the fertile window at a ratio of 86%. This indicates that the test strip according to the present invention can adequately function as a biomarker for predicting the day of ovulation.
(3) The surge intensity was measured by comparing the day of the surge with the day before surge. As a result, the leukorrheal LH showed a sensitivity of 85% and a specificity of 77% at a threshold of 0.15 mIU/mg. Furthermore, ROC analysis indicated that the AUC was 0.79. FIG. 6 is a graph showing the LH concentrations around the day of the leukorrheal LH surge. FIG. 7 is a graph showing the results of ROC analysis.

Discussion & Conclusion

Since the leukorrheal LH surge appeared in the fertile window at a ratio of 86%, it can adequately function as a biomarker. The leukorrheal LH surge was significantly intense and demonstrated a distinguishing capability with a sensitivity of 85% and a specificity of 77% when an appropriate measurement system was constructed.

This study shows the biological potential of a wearable system for measuring leukorrheal LH, and it is believed that the development of a testing device will increase the chances of women attempting to conceive being able to become pregnant with reduced burden.

<Summary of Results>

It is important for women hoping to become pregnant to identify the day of ovulation in advance. Currently, there are various methods for predicting the day of ovulation, such as direct observation using ultrasound, basal body temperature, and fern crystal observation, but the most widely used method is a test for luteinizing hormone (LH) in urine from the viewpoint of convenience, etc. Though the measurement of urinary LH is relatively convenient, as it can be completed in about 15 minutes after applying urine to a test kit, daily measurement is still a heavy burden on the user, and the utilization rate of urinary LH test kits among women attempting to conceive is not very high. Thus, our research group focused on leukorrhea as a specimen. Not only can leukorrhea be sampled non-invasively, but it has an advantage of minimizing the burden on the user, as conscious sampling thereof is not necessary. However, leukorrheal LH has not been thoroughly studied, and it was unclear how much LH is contained in leukorrhea and whether its behavior is sufficient to predict the day of ovulation. In this test, as described above, urine and leukorrhea from 29 subjects over 10 day-period, from 10 to 19 days after the start of menstruation were collected, and LH in urine and in leukorrhea of the subjects was measured and compared by ELISA. As a result, an LH surge similar to that in urine was observed in leukorrhea, and assuming that the urinary LH surge identified by the urinary LH test kit is 1 day before ovulation, the ratio of leukorrheal LH surge appearing "5 days before ovulation to 0 days before ovulation", when the probability of pregnancy is high, was 86% (Table 1 above). Furthermore, it was discovered that the amount of leukorrheal LH was less than that of urinary LH, and thus a detection system with higher sensitivity than that of commercially available urinary LH test kits is required for detection. Though the results of this test do not necessarily indicate that urinary LH and leukorrheal LH behave in the same manner in terms of various characteristics such as concentration and surge day, it is considered that it is fully possible to predict the day of ovulation by measuring leukorrheal LH.

<<Examples 1 and 2>>

In Examples 1 and 2, as test strips according to the present invention, "Example 1" and "Example 2" were produced and their performances were examined.

(Standard Solution A)

The LH standard solution was a casein blocking buffer solution of LH. Specifically, the positive specimen solution was "Lumipulse Presto LH LH Calibrator (250 mIU)" (Fujirebio Inc.: Code No. 291573), and the dilution solution was 1% casein, 100 mM borate, pH 8.5. The positive specimen solution was diluted with the dilution solution to an arbitrary concentration to prepare the standard solution.

(Example 1)

The test strip of Example 1 comprised:

a first member which contacted vaginal discharge,
a second member which contained a labeled antibody that recognized luteinizing hormone in vaginal discharge, and
a third member which had a display part which contained a capture antibody,
the capture antibody was capable of capturing a complex formed by binding of the labeled antibody and luteinizing hormone, and
the test strip was configured such that the luteinizing hormone in vaginal discharge moved from the first member, through the second member, to the display part of the third member, and that
the display part of the third member changed color when the capture antibody captured the complex.

(Example 1 (Spec 1))

The test strip of Example 1 had a length of 12 mm and a width of 3 mm. The sample pad as the first member had a thickness of 0.1 mm, the conjugate pad as the second member had a thickness of 0.55 mm, and the membrane as the third member had a thickness of 0.1 mm, with the sum of the thicknesses of the first to third members being 0.75 mm. The average fiber diameter of the fibers constituting the first member was approximately 2 μm, and the average fiber diameter of the second member was approximately 20 μm. The labeled antibody in the conjugate pad contained red nanobeads having a particle diameter of 335 nm (product name: NanoAct, manufactured by Asahi Kasei Corporation) as a colorant.

The average fiber diameters of the fibers constituting the first member and the second member were obtained by measuring the fiber diameters of a sample cut to a predetermined size and set it in a scanning electron microscope (FlexSEM1000, manufactured by Hitachi High-Tech Corporation), and averaging the measured fiber diameter values of N=30 or more.

(Example 2 (Spec 2))

The dimensions of the test strip of Example 2 and the dimensions of the first to third members were the same as those of Example 1. The labeled antibody in the conjugate pad of Example 2 contained blue nanobeads having a particle diameter of 320 nm (product name: NanoAct, manufactured by Asahi Kasei Corporation) as a colorant.

Furthermore, the labeled antibodies used in Examples 1 and 2 differed in the manner in which they recognized LH. LH is a glycoprotein composed of two subunits $\alpha$ and $\beta$, and these subunits separate after the LH surge. The antibody used in Example 1 was capable of binding only to the assembly where the $\alpha$ subunit and $\beta$ subunit were bound together ($\alpha$-$\beta$), whereas the antibody used in Example 2 was capable of binding not only to the $\alpha$-$\beta$ assembly where the $\alpha$ subunit and $\beta$ subunit were bound together ($\alpha$-$\beta$) but also to the $\beta$ subunit.

Furthermore, in Example 2, as compared to Example 1, blue, which was easily visible even if it was faint, was used, and the amount of labeled antibody was doubled.

(Sensitivity Test)

Standard solutions containing various concentrations of LH were each dropped twice (each 24 $\mu$L) onto the sample pads of the test strips of Example 1 and Example 2, and after thirty minutes, the color change at the display parts was observed. The difference of Example 2 from Example 1 was that the detection accuracy was improved and the visibility was also improved, and thus the probability that the wearer could judge by themselves was improved.

The results are shown in FIG. 1.

As can be seen from FIG. 1, in Example 1, when the LH concentration was 0.5 or 1.0 mIU/mL or more, a color change at the display part was observed.

Regarding Example 1, a proportional relationship between the LH concentration and the color strength at least in the concentration range of 0.5 to 1 IU/mL was observed.

Furthermore, as can be understood from FIG. 1, in Example 2, when the LH concentration was 0.05 or 0.1 mIU/mL or more, a color change at the display part was observed.

In Example 2, a proportional relationship between the LH concentration and the color strength at least in the concentration range of 0.05 to 1.0 mIU/mL was observed.

<Comparison>

The test strips of Examples 1 and 2 were compared in terms of color intensity.

The results are shown in FIG. 2. As can be seen from FIG. 2, the test strip according to the present invention showed good color intensity over a wide range of LH concentrations. The strip (Example 2) of Example 2 showed better color intensity than the strip (Example 1) of Example 1.

<<Examples 3 and 4>>

In Examples 3 and 4, the relationship between the width of the test strip and the visibility of the color change was examined. The test strip of Example 3 had a width of 5 mm, and the test strip of Example 4 had a width of 3 mm. The width of the test strip means the length in the overall plane of the test strip in the direction perpendicular to the length of the test strip (in particular, the direction in which the vaginal discharge travels).

Vaginal discharge sampled from each of the three subjects over 10 to 12 days was allowed to adhere to the sample pad of the test strip of Example 3 or 4, which had a similar configuration as in Example 2 described above, and the color change at the display part was observed. The total number of tests performed was n=25 for Example 3 and n=28 for Example 4.

Regarding the color change at the display part, the ratio of cases in which the control line was clearly visible (Excellent) and cases in which the control line could be visually confirmed but with slightly poor visibility (Poor) was calculated. The results are shown in Table 2 below.

[Table 2]

| | Test strip width | Total sample number (n) | Visibility evaluation | | | |
|---|---|---|---|---|---|---|
| | | | Excellent | Poor | Ratio of excellent | Ratio of poor |
| Example 3 | 5 mm | 25 | 16 | 9 | 64% | 36% |
| Example 4 | 3 mm | 28 | 24 | 4 | 86% | 14% |

As can be seen from Table 2, the test strip of Example 4, which had a width of 3 mm, exhibited better visibility than the test strip of Example 3, which had a width of 5 mm.

Without intending to be limited by theory, it is believed that the flow of the sample was improved by the relatively narrow width of the test strip. Specifically, it is believed that the test strip of Example 4 had improved visibility as a result of a relatively increased amount of vaginal discharge (and its components) components reaching the display part, which was the color-changing area.

## Claims

**1.** A test strip for detecting luteinizing hormone in vaginal discharge using immunochromatography, wherein 0.05 mIU/mL to 2.5 mIU/mL of luteinizing hormone is detected as positive.

**2.** The test strip according to claim 1, wherein the test strip has a width of 5 mm or less.

**3.** The test strip according to claim 1, wherein the test strip has a width of 3 mm or less.

**4.** The test strip according to claim 1 or 2, wherein the test strip comprises:

a first member which contacts vaginal discharge,
a second member which contains a labeled antibody that recognizes luteinizing hormone in vaginal discharge, and
a third member which has a display part which contains a capture antibody,
the capture antibody is capable of capturing a complex formed by binding of the labeled antibody and luteinizing hormone, and
the test strip is configured such that the luteinizing hormone in vaginal discharge moves from the first member, through the second member, to the display part of the third member, and that
the display part of the third member changes color when the capture antibody captures the complex.

**5.** The test strip according to claim 4, wherein the first member, the second member, and the third member are stacked in this order at least partially overlapping each other, and
a sum of thicknesses of the first member, the second member, and the third member in the stacking direction is 1.5 mm or less.

**6.** The test strip according to claim 5, for use in direct contact with the vagina.

**7.** The test strip according to claim 4, which is configured such that a top side and a back side of the test strip can be visually distinguished.

**8.** The test strip according to claim 4, wherein the first member is composed of fibers having an average fiber diameter of 0.5 to 5 $\mu$m,

the second member is composed of fibers having an average fiber diameter of 10 to 40 $\mu$m, and
an average fiber diameter of the fibers of the first member is less than an average fiber diameter of the fibers of the second member.

**9.** The test strip according to claim 4, wherein an average fiber diameter R1 of the fibers constituting the first member and an average fiber diameter R2 of the fibers constituting the second member satisfy the relationship $5 \leq (R2/R1) \leq 20$.

**10.** The test strip according to claim 4, which satisfies at least one of the following:

(a) the second member has a basis weight of 100 gsm or more,
(b) a sum of thicknesses of the first member, the second member, and the third member is 1.5 mm or less, and the thickness of the second member accounts for 30% or more of this sum of the thicknesses, and
(b) the second member has a density of 0.05 to 0.80 g/$m^3$.

**11.** The test strip according to claim 4, wherein the labeled antibody comprises a colorant having a particle diameter of 10 nm to 500 nm.

**12.** The test strip according to claim 4, wherein the labeled antibody comprises a colorant having a particle diameter of 250 nm to 500 nm.

**13.** The test strip according to claim 4, wherein a surfactant is applied to the first member, which is a sample pad.

**14.** The test strip according to claim 4, wherein the first member, which is a sample pad, contains a boron compound and a lectin.

Fig. 1

EXAMPLE 1

(mIU/mL)

0 0.05 0.1 0.2 0.5 1.0

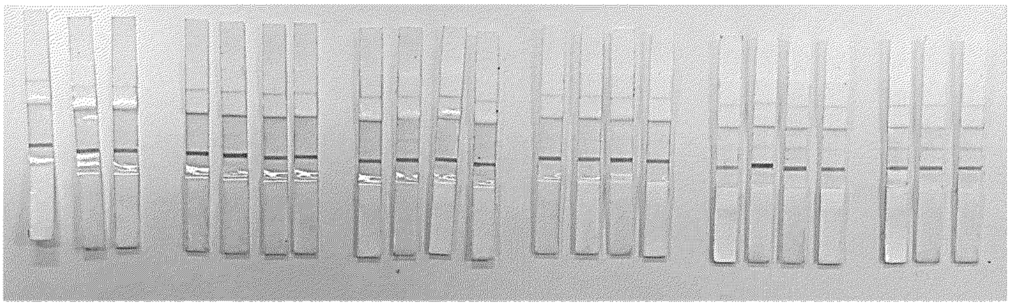

EXAMPLE 2

(mIU/mL)

0 0.05 0.1 0.2 0.5 1.0

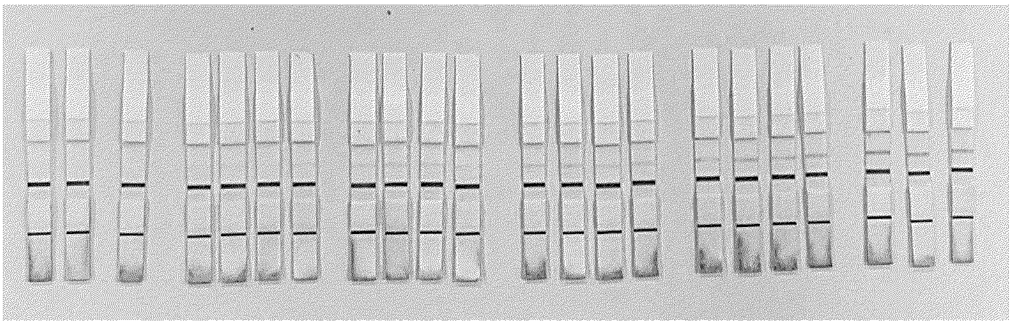

Fig. 2

| Sensitivity[mIU] | 0 | 0.05 | 0.1 | 0.2 | 0.5 | 1 |
|---|---|---|---|---|---|---|
| EXAMPLE 1 | 11.1 | 15.8 | 33.3 | 24.5 | 41.2 | 103.2 |
| EXAMPLE 2 | 13.8 | 26.9 | 41.1 | 60.7 | 85.0 | 177.0 |

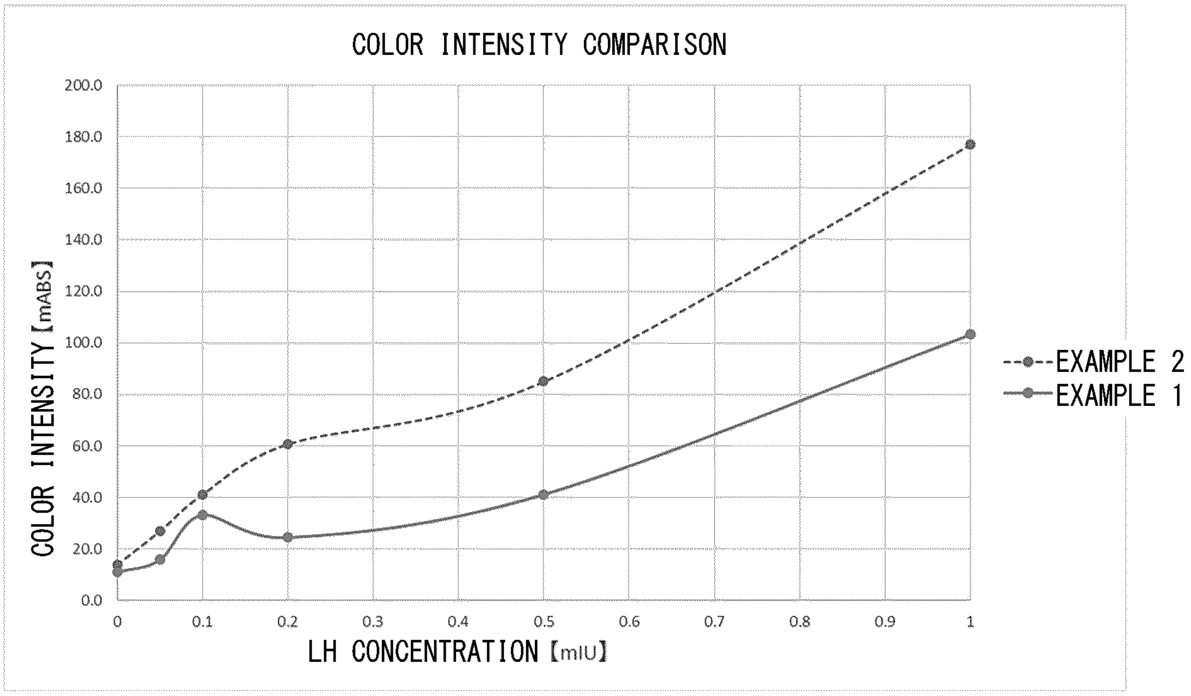


Fig. 3-1

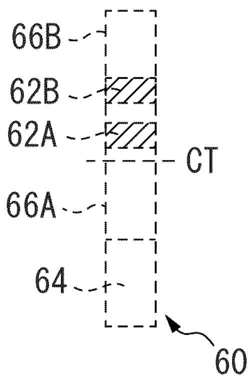

Fig. 3-2

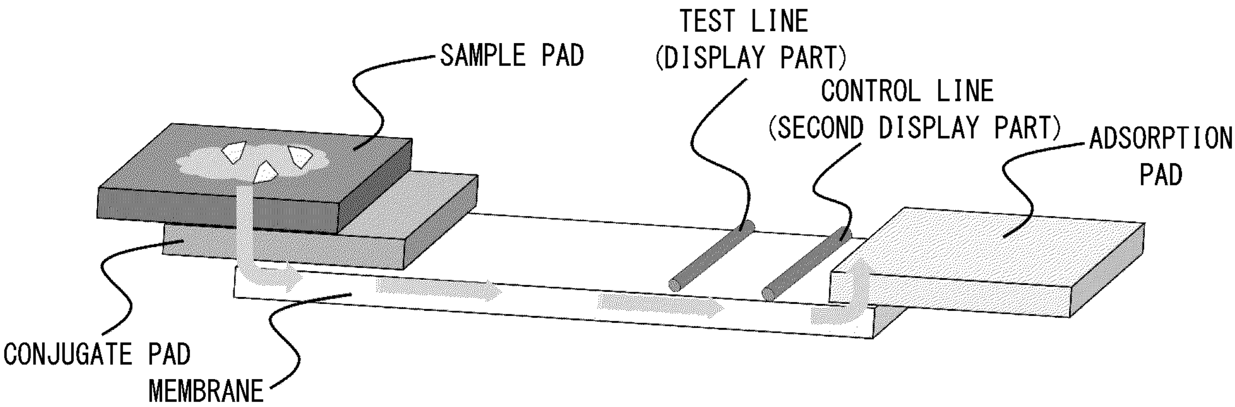


Fig. 4

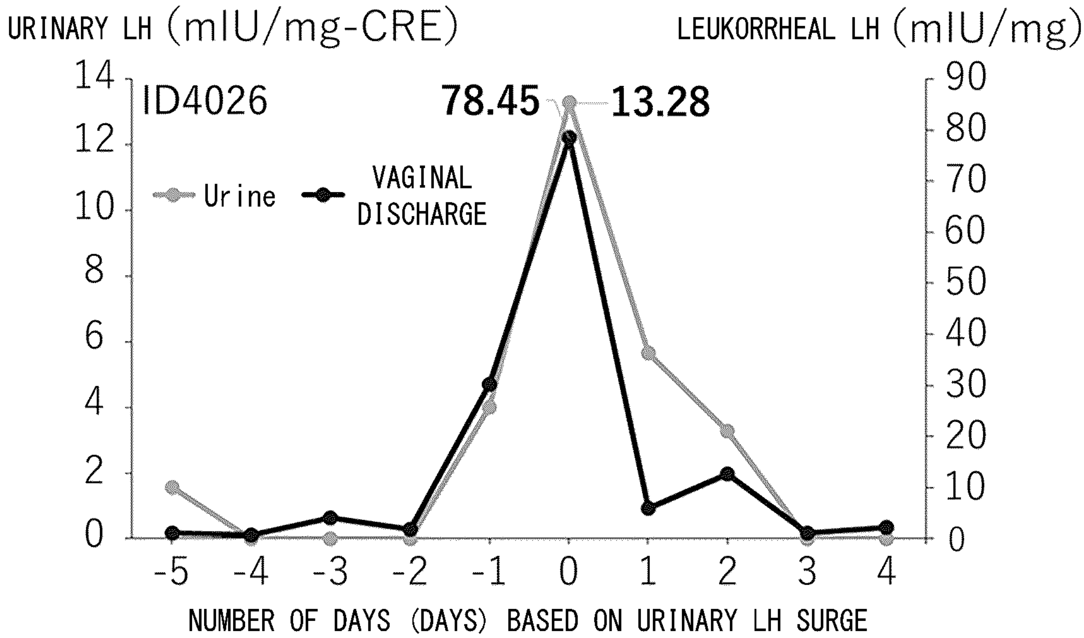


LEUKORRHEAL LH AND URINARY LH SURGED ON THE SAME DAY

Fig. 5

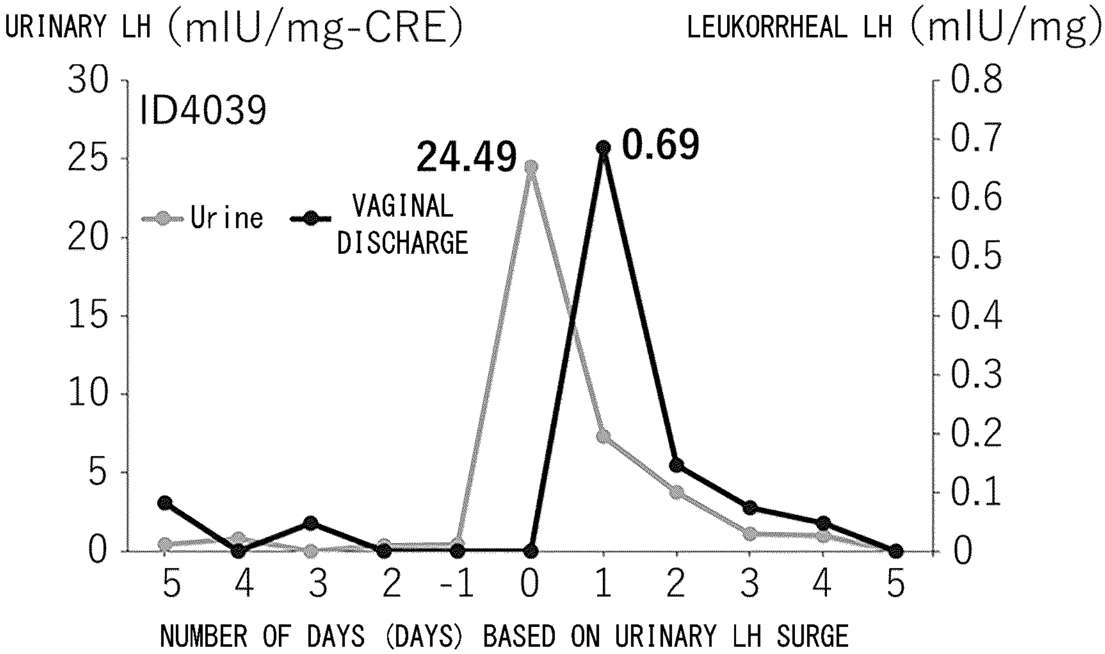


LEUKORRHEAL LH SURGED 1 DAY LATER THAN URINARY LH

Fig. 6

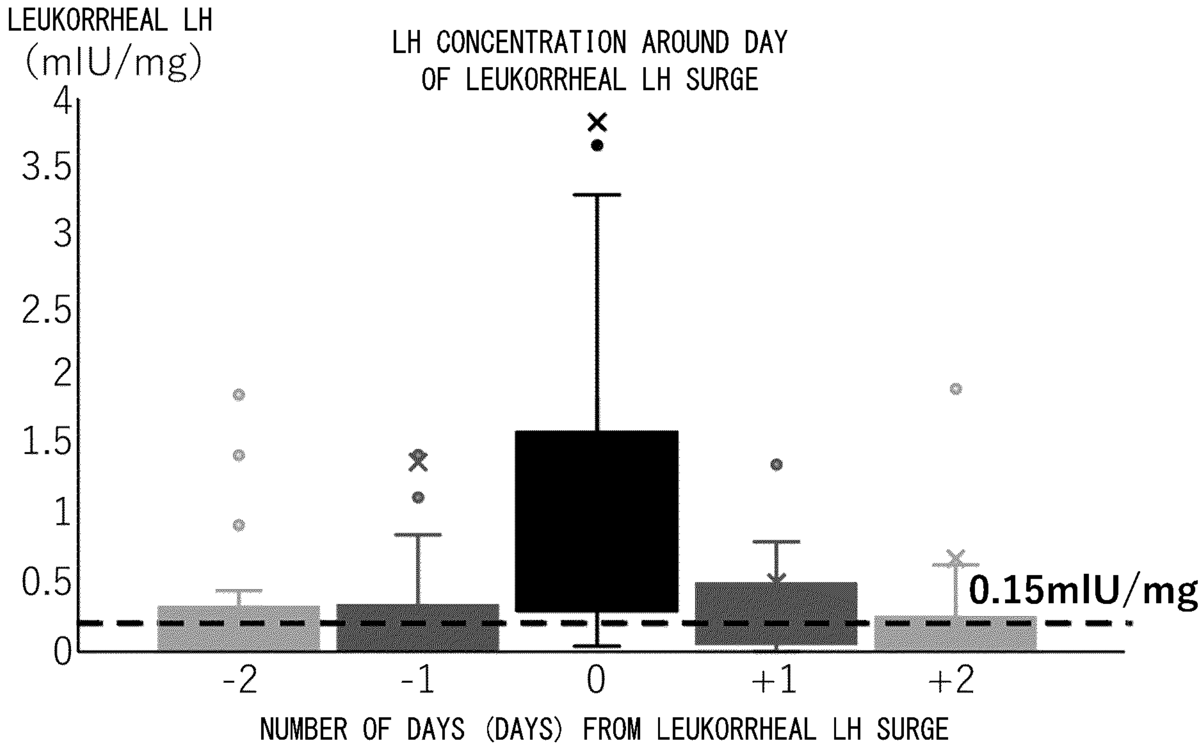

Fig. 7

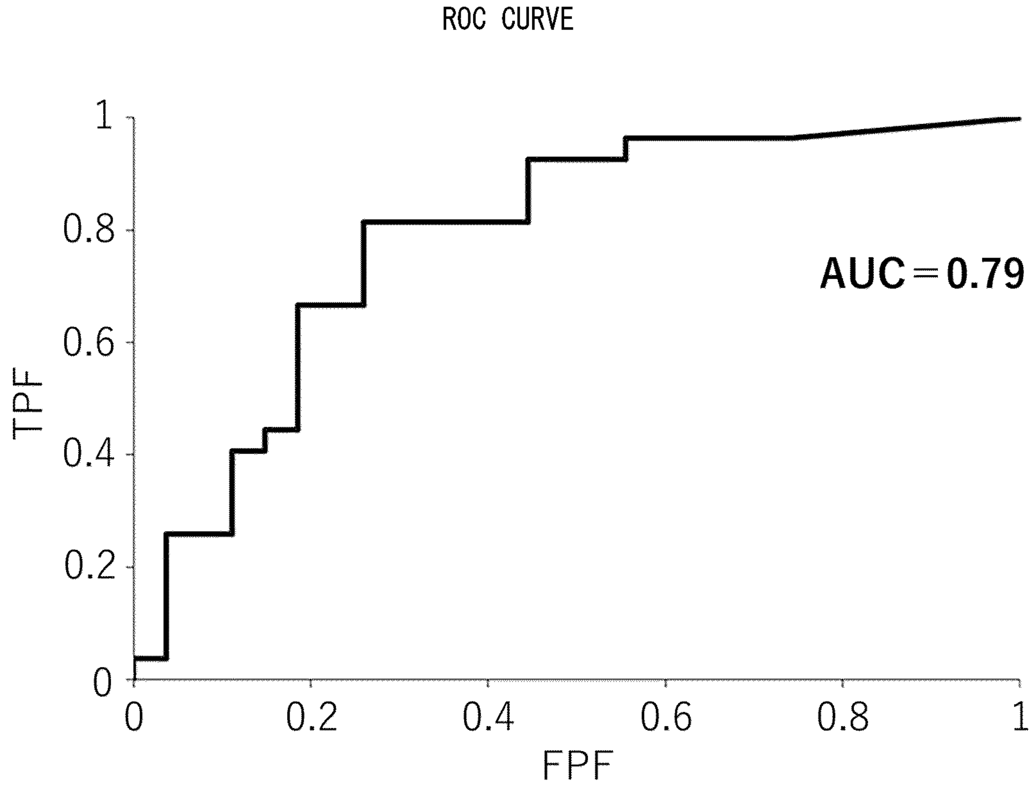

**INTERNATIONAL SEARCH REPORT**

International application No.
**PCT/JP2023/037939**

**A. CLASSIFICATION OF SUBJECT MATTER**

***G01N 33/53***(2006.01)i; ***G01N 33/543***(2006.01)i
FI: G01N33/53 B; G01N33/543 521

According to International Patent Classification (IPC) or to both national classification and IPC

**B. FIELDS SEARCHED**

Minimum documentation searched (classification system followed by classification symbols)

G01N33/53; G01N33/543

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2021/132724 A1 (UNICHARM CORP.) 01 July 2021 (2021-07-01) paragraphs [0048]-[0060], [0081], [0082], fig. 1, 3, 6 | 1-14 |
| A | WO 2016/002743 A1 (NIPPON STEEL & SUMITOMO METAL CORP.) 07 January 2016 (2016-01-07) paragraphs [0005]-[0026] | 1-14 |
| A | JP 8-285849 A (MOCHIDA PHARMACEUT. CO., LTD.) 01 November 1996 (1996-11-01) paragraphs [0022]-[0025], [0064]-[0067], [0089]-[0094] | 1-14 |
| A | WO 2020/053966 A1 (UNICHARM CORP.) 19 March 2020 (2020-03-19) paragraphs [0033]-[0050] | 1-14 |
| A | JP 2003-517584 A (THE PROCTER & GAMBLE CO.) 27 May 2003 (2003-05-27) entire text, all drawings | 1-14 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/037939**

| Patent document cited in search report | Publication date (day/month/year) | Patent family member(s) | Publication date (day/month/year) |
|---|---|---|---|
| WO 2021/132724 A1 | 01 July 2021 | CN 114845674 A<br>entire text, all drawings | |
| WO 2016/002743 A1 | 07 January 2016 | US 2017/0168049 A1<br>entire text, all drawings<br>EP 3165923 A1<br>CN 106662585 A | |
| JP 8-285849 A | 01 November 1996 | (Family: none) | |
| WO 2020/053966 A1 | 19 March 2020 | CN 112672721 A<br>entire text, all drawings<br>KR 10-2021-0041050 A | |
| JP 2003-517584 A | 27 May 2003 | JP 2002-542843 A<br>entire text, all drawings<br>WO 2000/000233 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007526443 A
- WO 2021132724 A1


### Non-patent literature cited in the description


- *Bioeng. Transl. Med.*, 2017, vol. 2 (3), 238-246
- Monitoring the Menstrual Cycle: Comparison of Urinary and Serum Reproductive Hormones Referenced True Ovulation. *The European Journal of Contraception and Reproductive Health Care*, 2015
- Characteristics of Urinary Luteinizing Hormone Surge in Young Ovulatory Women. *Fertil. Steril.*, September 2007, vol. 88 (3), 684-90
- **D. B. DUNSON et al.** Day-Specific Probabilities of Clinical Pregnancy Based on Two Studies with Perfect Measurements of Ovulation. *Human Reproduction*, 1999, vol. 14 (7), 1835-1839